(19) **Europäisches Patentamt**

**European Patent Office**

**Office européen des brevets**

(11) Publication number: **0 283 443 B1**

(12) **EUROPEAN PATENT SPECIFICATION**

(45) Date of publication of patent specification: **30.06.93**

(51) Int. Cl.⁵: **C07K 3/02**, C07K 15/06, C07K 15/16, A61K 39/00

(21) Application number: **88830058.9**

(22) Date of filing: **17.02.88**

(54) **Process for extracting membrane antigens having immunogenic properties from human neoplastic cells and products thereby obtained.**

(30) Priority: **18.02.87 IT 4765887**

(43) Date of publication of application:
**21.09.88 Bulletin 88/38**

(45) Publication of the grant of the patent:
**30.06.93 Bulletin 93/26**

(84) Designated Contracting States:
**AT BE CH DE ES FR GB GR LI LU NL SE**

(56) References cited:
**EP-A- 0 132 281**

**JOURNAL OF BIOLOGICAL RESPONSE MODI-FIERS, vol. 5, no. 3, June 1986, pages 211-224, Raven Press, New York, US; J. C. BYSTRYN et al.: "Preparation and characterization of a polyvalent human melanoma antigen vaccine"**

(73) Proprietor: **ISTITUTO FARMACOTERAPICO ITALIANO S.P.A.**
**Via Paolo Frisi 21/23**
**I-00197 Rome(IT)**

(72) Inventor: **Tarro, Giulio**
**Via Posillipo, 286**
**I-80123 Napoli(IT)**

(74) Representative: **de Simone, Domenico et al**
**Ing. Barzanò & Zanardo Roma S.p.A. Via Piemonte 26**
**I-00187 Roma (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

The present invention relates to a process for extracting membrane antigens having immunogenic properties from human neoplastic cells and the products thereof.

More particularly, the present invention relates to a process for extracting antigenic protein complexes that are capable of inducing delayed hypersensitivity and specific blastization of lymphocytes starting from human neoplastic material, as well as to the lipoglycoproteins so obtained to be employed in the specific immunotherapy of tumoral diseases.

As is well known, the oncologic research has been directing its efforts for more than a decade towards the problem of immunotherapy of tumoral diseases, on the basis of the reasonable possibility of finding a therapeutically useful solution through the manipulation of the immuno-oncolytic reaction that the human organism can develop spontaneously. The initial urge towards such trend of the scientific research can be recognized in the first observations (I.S. Irlin, Virology, 1967, 32:725, E. Klen et al., Nat. Cancer Inst., 1964, 32:547, G.J. Pasternak, J. Nat. Cancer Inst., 1965, 34:71, S.S. Tevethia et al., Immunol., 1968, 100:358, R. Nishioka et al., Monograph, 1968, 7:49) regarding the stimulation of specific humoral and cellular antibodies by the antigens of neoplastic cells both in animals and in human beings.

The immune manipulations attempted so far as an immunotherapeutic approach, while reflecting the knowledge successively acquired concerning the physiopathology of cancer and of the immune system of the host, on the other side also suffers from the lack of suitable immunogenic agents or from the difficulty experienced in removing the situations of block of cellular immunity which are present in the cancer patient.

As a matter of practice, two main roads have been followed:

a) the non-specific activation of the host's immunity in order to strengthen the immuno-oncolytic reactions (immuno-adjuvants),

b) the specific activation of the host's immunity in order to electively stimulate the production of antibodies having oncolytic effects.

Actually, up to recent times only the first pattern (the non-specific activation) had been followed in a quite widespread manner, with acceptable results, whereas the second one (the specific activation) had remained just an experience at the theoretical level, owing to the difficulty of obtaining effective specific antigens from the neoplastic cell (the syntheses for the production of such antigens were unsuccessful).

With no doubt a large technical literature exists (D.L. Morton et al., Annals of Internal Medicine 74:587-604, 1971, G. Mathé, Presse Medicale 75:947, 1967, C. Chyba et al., Arch. Int. Med. 115:558, 1965, J.W. Finney et al., Cancer Res. 20:351, 1960, E. Guidetti, La clinica terapeutica vol. 44, 6, 1968, A. Haddow et al., The Lancet 29/2, 452, 1964, R.L. Ikonopisov et al., Brit. Med. J. II, 752, 1970) concerning the research carried out in such field, which however was not followed by therapeutical applications, owing above all to the face that antigens extracted from autologous or homologous neoplastic cells did not show immunogenic, even though the results obtained seemed to direct the experimentation in that sense.

Putting aside as unsuitable for application the alternative of the cellular immune response in which the lymphocytes T are sensitized to the response by links of the antigens with their receptors through macrophages, the alternative remained of the humoral immune response, and indeed the latest experimentation is directed towards this alternative.

However, the problem remained of the possibility of having at disposal a material suitable to induce through that way a correct immune response, as the antigens extracted from the tumoral tissue failed to reach the lymphocytes in such conditions or in sufficient number to activate the same. This is especially due to the barrier or shelter that forms on the cell walls when the antigens become fixed to the corresponding receptors giving rise to immunocomplexes. Indeed, in such conditions the immunocomplexes become "sequestered" on the cell wall and they cannot penetrate the same as it is desired.

The teaching of the present invention is directed to an efficient immunotherapy of tumoral diseases which provides the same basic operations of the prior art, consisting in:

a) extracting antigens from the tumour, and

b) stimulating by means of such antigens the lymphocyte clone thus inducing a response of "allergic" type for the rejection of the tumour,

but without the drawbacks of the similar technologies already known which showed unapplicable. Indeed, the operation was unsuitable to be carried out correctly as the antigens extracted were not at all or not enough immunogenic, above all because of their small sizes, their faulty stimulating capacity, or because of the incapability of "coupling" with the tumoral cell.

Substantially, the problem to solve was to supply the lymphocytes (lymphocytes T) with the information necessary to have at disposal said "coupling means" or "keys" of such nature as to make them operative without the aid of adjuvants as provided by the schemes of the known technologies.

Accordingly, the necessity and the importance of having at disposal antigens like the antigenic protein complexes of the invention are quite evident, such complexes being defined in the following as TLP (tumour liberating proteins). Such antigens are of larger sizes, and capable of stimulating the lymphocytes T so as to cause them to be active as regards the tumoral aggression, through a delayed immune response.

Attempts at satisfying such necessity have already been performed by means of specific extractive procedures, like those completed by Prof.G. Tarro and Prof. A. Pederzini, which are summarized in the articles published in the journal "Oncology" starting from 1982.

In a comparison with such technologies, in order to put into evidence the innovations of the procedure which is the object of the present invention, it is to keep in mind the remarkable length of their operative path. In such technologies the starting tumoral matter, suitably minced, is to be subjected to:

a) a "collector system" through a molecular sieve of 60 mesh stainless steel with previous centrifugation at 15,000 × g,

b) ultracentrifugation at 100,000 × g,

c) filtration through Sephadex, ionic exchange chromatography and affinity chromatography.

Said "collector system" gave rise to material losses, such material being kept on the sieve meshes, and that also the operation of previous centrifugation at 15,000 × g affected negatively the yield.

The purification operations which characterize the extraction procedure mentioned above are made necessary and uneliminable by the fact that in step (b) not only the membrane proteins like those desired are recovered, but also endocellular and exocellular proteins, and their presence calls for a successive complicated purification step (c).

In view of that, a later extraction procedure (by A.C. Hollinshead) is directed to a selective previous purification of the membrane antigenic proteins through the successive employment of isotonic and hypotonic solutions and of a series of sonications, so as to simplify the next purification operation through filtration. However, this procedure is all the same a complex and burdensome technique, which is not always satisfying.

Thus, according to the present invention, an alternative prof extraction of the TLP proteins is proposed, in which procedure the drawbacks mentioned above are removed through an improved purification operation that includes isoelectric focussing (IEF), in addition to a previous separation technique through centrifugation with a higher number of operative cycles wherein the successive supernatants are collected to form the definitive pool.

The present invention specifically relates to a process for extracting, from human neoplastic cells, membrane antigens having immunogenic properties and capable of inducing delayed hypersensitivity and the specific blastization of lymphocytes, in which process the neoplastic tissue, after removal of the necrotic material and after washing in PBS (Phosphate Buffer Solution), is minced and homogenized (as for instance in a Waring Blendor) with repeated coolings in ice, and the homogenate after threefold freezing and thawing is subjected to sonication, said process being characterized in that it further comprises the successive operations of:

a) subjecting the sonicated matter to a previous centrifugation at 3,000 × g according to the following scheme:

```
           Previous centrifugation at 3000 x g


         ┌  Supernatant (1)    ' Sediment (1)          ┌              ┐
         │                              │              │ homonogeniz- │
         │                              │              │ ation, soni- │
         │                              ▼              │ cation, cen- │
    Pool │  Supernatant (2)     Sediment (2)           │ trif. 3000xg │
         │                              │              └              ┘
         │                              │
         │                              ▼
         └  Supernatant (3)     Sediment (3)  (to be discarded)
```

in which the sediment (1) from the first previous centrifugation, after homogenization, sonication and further centrifugation at 3,000 x g gives rise to the supernatant (2) and to the sediment (2), and the latter, subjected to the same procedure, forms the sediment (3), which is discarded, and the supernatant (3), which in added to the supernatants (1) and (2) to form a pool,

b) centrifuging said pool of the supernatants (1), (2) and (3) at 100,000 x g and concentrating the supernatant,

c) introducing, after activity test, the supernatant into an isoelectric focussing (IEF) column and collecting the peak fractions, checking their activity, and

d) increasing the purification degree by treatment in a chromatographic column.

Preferably, according to the present invention, the sonication is carried out in ice at 0.9 Kc and the centrifugation of the pool of the supernatants is carried out at 100,000 x g for 60', the full procedure being performed at 4°C in a sterilized environment.

Advantageously, the concentration of the final supernatant occurs by filtration on sucrose and/or other filtering aids such as polyacrylamide, Sephadex etc., and said isoelectric focussing is performed at a pH of 3.5-10, the final purification step through chromatography being carried out in a DEAE A (Sepharose) column.

The physical properties of the extracted material are preferably determined on a gel of IEF column and on SDS PAGE/Sephadex as regards molecular weight and glycosidic groups.

The antigenic protein products (TLP) obtained according to the present invention are substantially lipoglycoproteins having the following distinctive characteristics:

a) sizes between 4.81 and 6.1 nm (48.1 and 61 Angstrom) (on the basis of molecular weight data known),

b) presence of a lipidic group (on the basis of elution on concanavaline A),

c) solubility (undeterminable hypothetically as both density and the sedimentation coefficient are unknown) and

d) isoelectric point less than 7.0 (on the basis of the elution characteristics on Sephadex DEAE A (1 per anion) (elution with 0.2 M NaCl)).

Thus, the present invention provides antigenic protein complexes (TLP) having the physical properties mentioned above which, as said before, if injected intradermally (employing millipore filters) into the same patients from which the neoplastic material had been derived (autologous TLP) or into other patients bearing neoplasias of the same histological type (homologous TLP), were shown to develop a therapeutic activity efficient towards the tumoral disease.

Some experiments are reported in the following for illustrative and not for limitative purposes, said experiments being carried out with the above-metioned TLP antigens and being followed by an estimate of the results obtained.

Experiment 1

Intradermal injections were made in the cutis of the deltoid regions with autologous and homologous TLP, with histological study of the reaction induced in the injection point and of the behaviour of some immunologic parameters after the injection of the antigen.

Patients suffering from cancer were chosen for this research who had been shown immunologically active in some previously performed immunologic monitoring tests.

The skin reaction induced by the intradermal injection of TLP was characterized by the appearance on the second day of a hyperemic nodular manifestation of the approximate sizes of a lentil which persisted for some time, even for months, according to the case, and which could extend up to the central necrosis.

Biopsy of samples at the points of such nodular reactions, taken on the fifth-sixth day, showed lymphomonocyte infiltration,which is typical of the delayed hypersensitivity reaction.

Experiment 2

The study of the mitogenic activity was performed on cultured peripheral lymphocytes taken from healthy subjects, from patients suffering from malignant neoplasia of the same histological type as that of the tumour from which the TLP had been extracted, and from patients suffering from malignant neoplasia of the same histological type as the tumour TLP was extracted from, but who had been pretreated with TLP intradermally.

The method of study of the mitogenic activity of TLP in vitro employed is the cytomorphologic method according to the procedure disclosed by Astaldi et al..

The results concerning the blastizing activity of TLP in the various groups of subjects under examination: healty subjects, cancer patients, cancer patients pretreated with TLP are summarized hereinbelow, with the levels of induced blastization.

From the results obtained it is possible to remark that:

- cancer patients inoculated with TLP obtained from their own neoplastic tissue (autologous TLP) and also cancer patients inoculated with TLP obtained from another patient's tumor, but with the same histological diagnosis (homologous TLP) constantly show a delayed reaction having the histological characters typical of the reaction of delayed hypersensitivity. It is to be stressed that the reaction is induced without the aid of Freund's adjuvant;
- the TLP tested on peripheral lymphocytes of healthy subjects does not induce blastization of the lymphocytes;
- the TLP tested on peripheral lymphocytes of patients suffering from cancer with the same histological diagnosis as that from which said TLP is derived seems not to induce blastization of lymphocytes (at least in the limited number of cases considered so far);
- the TLP tested on peripheral lymphocytes of patients suffering from tumour with the same histological diagnosis as that from which the antigen is derived (autologous and homologous) and to whom the antigen had been previously repeatedly injected, induces the blastization of lymphocytes.

From the whole set of the observations collected the following conclusions can be drawn:

a) the TLP seems not to have non-specific mitogenic activity as it does not blastize the lymphocytes of healthy subjects and of patients suffering from cancer who have not been pretreated with the same antigen;

b) the TLP seems on the contrary to be endowed with specific mitogenic activity; the blastization of lymphocytes induced in cancer patients previously treated means that the TLP, when injected intradermally, is capable of sensitizing a limphocyte clone which becomes quickly blastized when it meets again the antigen in vitro.

Accordingly, it appears that the TLP, being immunogenic, specifically mitogenic, free from negative secondary immunologic reactions (there were no observations of atopic manifestations, either auto- or homoimmunopathic, and of Arthusian phaenomena), and also being an element of the antigenic endowment of the neoplastic cell, is a valid basis for an approach to the specific immunotherapy of cancer.

Such conclusive statements can be adequately supported by the practical results of the experiments reported in the following tables 1-5.

Table 1

TLP-blastization of peripheral lymphocytes of different groups of carcinomata

| Type of carcinoma | No. of cases | Skin reaction | Basic blastization homologous TLP | Basic blastization autologous TLP |
|---|---|---|---|---|
| Bronchial adenocarc. | 21 | 17/21 | 6/21 | |
| Breast adenocarc. | 10 | 10/10 | **3/9** | 1/1 |
| Big intest. adenocarc. | 10 | 7/10 | 1/10 | |
| Bladder adenocarc. | 1 | 1/1 | 0/1 | |
| Fibromyosarcoma | 1 | 1/1 | | 0/1 |
| Uterus carc. | 1 | 1/1 | 1/1 | |

6

| | | | | |
|---|---|---|---|---|
| Gastric carc. | 1 | 1/1 | 0/1 | |
| Ovary carc. | 2 | 1/2 | 0/2 | |
| Melanoma | 1 | 1/1 | 1/1 | |
| Vulva spinocel. carc. | 2 | 2/2 | 1/2 | |
| Tongue spinocel. carc. | 1 | 1/1 | 0/1 | |
| Total | 51 | 43/51 80 % | 13/49 26 % | 1/2 |

Table 2

Induction of lymphocyte blastization against TLP after repeated intradermal introduction of the same antigen

| Type of carcinoma | No of cases | Skin reaction | Isoneopl. homol. TLP | Autol. TLP | Basic blast. with TLP | Blast. after treatment with TLP |
|---|---|---|---|---|---|---|
| Bronchial adenocarc. | 11 | 8/11 | 11/11 | - | 3/11 | 8/8 |
| Big intest. adenocarc. | 11 | 8/11 | 11/11 | - | 1/11 | 5/6 |
| Breast adenocarc. | 10 | 10/10 | 9/10 | 1/10 | 4/10 | 5/5 |
| Bladder adenocarc. | 1 | 1/1 | 1/1 | - | 0/1 | 1/1 |
| Fibromyosarcoma | 1 | 1/1 | - | 1/1 | 0/1 | 1/1 |
| Uterus adenocarc. | 1 | 1/1 | 1/1 | - | 1/1 | - |

| | | | | | | |
|---|---|---|---|---|---|---|
| Gastric adeno-carc. | 1 | 1/1 | 1/1 | - | 0/1 | 1/1 |
| Ovary adenocarc. | 2 | 1/2 | 1/2 | 1/2 | 0/2 | 2/2 |
| Melanoblastoma | 1 | 1/1 | 1/1 | - | 1/1 | - |
| Vulva spinocell. carc. | 2 | 2/2 | 2/2 | - | 1/2 | 1/2 |
| TOTAL | 41 | 34/41 | 38/40 | 3/13 | 11/41 | 24/26 |

Table 3

Effect of chemical and physical cytostatic agents on the behaviour of lymphocyte blastization against TLP

| Carc. type | Homol. TLP | Autol. TLP | D.H.R. | Basic TLP blast. | Blast. after TLP treatm. | Cytost. treatment | Blast. after cytost. treatm. |
|---|---|---|---|---|---|---|---|
| Breast adeno-carc. | X | = | +++ | --- | +++ | Rx ter 6000 r/s | +-- |
| Breast adeno-carc. | X | = | +++ | --- | ++- | Rx ter 3000 r/s | --- |
| sigma adeno-carc. | X | = | ++- | ++- | ++- | Rx ter 600 r/s | --- |
| lung | X | = | +++ | --- | +++ | Rx ter | --- |

8

| | | | | | | | |
|---|---|---|---|---|---|---|---|
| adeno-carc. | | | | | | 4800 r/s | |
| Bronch. adeno-carc. | X | = | - - - | - - - | + + + | Vabem 3000 r/s | - - - |
| Bronch. adeno-carc. | X | = | + + - | - - - | + + + | Rx ter 600 r/s | - - - |

D.H.R. = Delayed hypersensitivity reaction.

## Table 4

Effect of the intradermal treatment with TLP on the peripheral
lymphocyte counting - Rosette E - Tests Cut.I.R.   B.I. → PHA   B.I. → TLP

| Carc. type | Basic TLP blast. | TLP blast. after TLP treatment | B.I. → PHA before-after TLP | | IGg before-after TLP | | P.L. Counting x mmc before-after TLP | | BCG before-after TLP | PDD before-after TLP | Rosette E before-after TLP |
|---|---|---|---|---|---|---|---|---|---|---|---|
| Bronch. adenocarc. 10 cases | 9/10 --- | 9/10 +++ | 9/10 27% | 39% | 10/10 942 IgG 202 IgA 130 IgM | 1802 136 138 | 10/10 2107 | 2043 | 10/10 ++- ++- | 10/10 ++-++- | 10/10 49%  47% |
| Breast adeno- carc. 6 cases | 6/6 --- | 6/6 +++ | 4/6 33% | 57% | 4/6 1578 239 182 | 999 199 182 | 6/6 1812 | 1453 | 6/6 ++- ++- | 6/6 ++- ++- | 6/6 45% 38% |
| Big intest. adenocarc. 7 cases | 6/7 --- | 6/6 +++ | 6/7 21% | 27% | 5/7 1580 242 138 | 1602 152 155 | 6/7 1662 | 1374 | 6/7 ++- ++- | ++- ++- | 6/6 52% 62% |
| | 21/23 | 21/21 | 19/23 | | 19/23 | | 22/23 | | 16/16 | 16/16 | 22/22 |

B.I. = Blastization index

Table   5

Induction of lymphocyte blastization
with TLP in cancer patients with dif-
ferent immunological expressions

| | | Blastization against TLP | |
|---|---|---|---|
| | | N of cases | % |
| ROSETTE E | Rosette E > 50% | 11/13 | 84 |
| | Rosette E < 50% | 10/12 | 83,3 |
| PPD TEST | PPD test +++ | 10/12 | 83,3 |
| | PPD test +-- | 6/6 | 100 |
| LYMPHOCYTES x mmc | Lymphocytes > 1500 | 9/12 | 75 |
| | Lymphocytes < 1500 | 5/6 | 83 |
| BI → PHA | BI → PHA > 40% | 12/12 | 100 |
| | BI → PHA < 40% | 3/8 | 38 |
| IgG | IgG > 1800 | 10/10 | 100 |
| | IgG < 1800 | 10/13 | 77 |

For illustrative and not for limitative purposes, the details of the IEF procedure adopted are disclosed in the following.

Isoelectric focussing (IEF):

1) column IEF.

The column employed for such type of resolution was the 8100-1 model of LKB (Bromma, Sweden) of 110 ml volume, the same as for ampholytes, all at a pH range of 3.5-10.

Solutions and procedure (Bishop R. Science Tools, The LKB Instruments 26:2; 1979; Abraham C.V. and Bakerman S. ibid. 24:22, 1977):

| a) The heavy solution: | |
|---|---|
| Sucrose<br>SN in water solution (total of 26.4 mg)<br>40% ampholytes | 28.0 g<br>31.5 ml<br>1.8 ml |
| b) The light solution | |
| $H_2O$<br>40% ampholytes | 42.0 ml<br>0.7 ml |
| c) Anodic solution, pH 1.2: | |
| sucrose<br>$H_2O$<br>1M $H_3PO_4$ | 15.0 g<br>12.0 ml<br>4.0 ml |
| d) The cathodic solution, pH 13.7: | |
| 0,25 M NaOH | q.s. to cover the electrode |

The column was loaded with the light and the heavy solution, that were caused to flow through a gradient mixer: thus a continuous sucrose gredient was formed (1-50% w/v) in which the ampholytes and the sample were distributed homogeneously. Then the separation under the action the electric field was started, such operation lasting 72 hours. The run conditions are reported in the following:

| | | V | W | mA |
|---|---|---|---|---|
| 0-17 hours: | initial values: | 1000 | 75 | 76 |
| 17-72 hours: | final values: | 2000 | 150 | 90 |
| | | 2000 | 150 | 10 |

The run always occurred at a temperature of 14 °C kept constant by a water jacket outside the column. At the end of the separation process the content of the column was collected by pressurization through a peristaltic pump at a rate of 110 ml/hour into 1.1 ml fractions whose pH value was immediately determined at 14 °C. Then the optical densities (O.D.) were determined at 280 nm. Finally the immunoenzymatic test was carried out in the fractions corresponding to the peak values, in order to estimate the possible immune activity of the sample.

2) IEF on a thin layer of polyacrylamide gel

The material that was shown to be immunoactive to column IEF was subjected to IEF on a thin layer of polyacrylamide gel.

The plate employed was of 1.5 × 120 × 200 mm size.

Solutions and procedure (Vesterberg O. Science Tools, The LKB Instruments 20:22, 1973; Vian M. and Constans J., ibid. 24:25, 1977; Bours J. ibid. 20:29, 1973):

| | |
|---|---|
| acrylamide (T = 6.6; C = 2.4) | 9.0 ml |
| 50% glycerol | 5.4 ml |
| 40% ampholytes | 1.8 ml |
| $H_2O$ | 10.8 ml |
| 2.2% ammonium persulfate | 0.2 ml |
| 5% sodium sulfite | 0.1 ml |

Stripes of Whatman 3 paper were employed as the cathode and the anode, said stripes being soaked respectively with 40% ampholytes and 1.0 M $H_3PO_4$.

The gel was subjected to a previous run of 20′ at a constant current of 90 mA. Then some small rectangular pieces of Whatman 3 paper were soaked respectively with: the immunoactive sample (C) and the negative control (see sect. 5) (C⁻).

The following scheme illustrates the distribution characteristics of the substances subjected to migration:

```
Cathode (-)

                              C⁻, C, Myo        Myo, C,

Anode (+)      Myo                                         Myo
```

As it can be remarked, the gel has been ideally divided into two halves along the cathode-anode line, in order to treat separately the two parts divided after the isoelectric migration. In the scheme above the presence can be observed of two Myo in opposition; thus, as myoglobin is a visible protein during the run because it is colored, it is possible to decide the stopping of migration at the very moment when the two bands are parallel at the same level.

The electrophoretic run occurring at a temperature of 14°C lasts 60′. The current strength decreases from the starting value of 30 mA to about 3 mA after 30′ run. The stability of such value shows in practice the end of the separation, which is confirmed by the same position reached by the two Myo bands in opposition.

Just at that time the gel is actually divided into two parts along the vertical line as shown in the scheme reported above. The part of the gel on the right is then subjected to the following procedures:

a) Fixing:
Acetylsalicylic acid: trichloroacetic acid: water = 5 : 5 : 90.
b) Washing:
Methanol: acetic acid: water = 45:10:45
c) Coloring:
0.02% Coomassie Brilliant Blue R 250 in methanol: acetic acid: water = 45:10:45
d) Washing:
Methanol: acetic acid: water = 45:10:45
e) Decoloring:
Methanol: acetic acid: glycerol: water = 22.5:7.5:5:5.65

A stripe corresponding to the migration path of C was cut longitudinally, in the sense of the run, from the part corresponding to that on the left of the scheme. Then said stripe was shredded into 35 slices along the minor section. Each of said slices was of an average length of 0.3 cm.

The slices ware dipped into small test tubes containing 0.5 ml of a physiologic solution and then the pH at 14°C of each of the solutions was determined. Then the slices were subjected to dialysis against 0.1 M carbonate-bicarbonate buffer at pH 9.8 and the immunoenzymatic test was carried out for estimating any possible immune activity of the eluate from the gel.

As regards the TLP, the results of some experimental tests are reported in the following.

Test of pyrogenic character

In order to check the presence in the TLP of substances which are possibly capable of inducing an increase in the temperature of the patients treated, a pyrogenic character test was performed. 20 $\mu$g of TLP are injected into three rabbits of 3 kg weight (exactly corresponding to the dose of 500 $\mu$g employed for men) and the rectal temperature was determined without remarking any increase in temperature during the whole day of check.

Sterility test

0.1 ml of TLP is poured onto Petri dishes containing agar-blood in order to check the development of bacterial colonies possibly present. Phenomena concerning the appearance of contaminations were never observed.

EP 0 283 443 B1

Safety test

According to the standard technology of the Food Drug Administration, solutions of 500 $\mu$g of TLP in 0.5 ml of water (corresponding to the human dose adopted in the therapeutical treatment) and respectively 1500$\mu$g/5 ml (exactly corresponding to the human dosage) were injected into two mice and two guinea pigs, without remarking any toxicity phenomenon in a period of 30 days.

The present invention has been disclosed with particular reference to some embodyments of the same, but it is to be understood that modifications and changes can be brought in without departing from its true spirit and scope.

## Claims

1. A process for the extraction, from human neoplastic cells, of membrane antigens having immunogenic properties and capable of inducing delayed hypersensitity and the specific blastization of lymphocytes, in which process the neoplastic tissue after removal of the necrotic material and washing in PBS (Phosphate Buffer Solution) is minced and homogenized with repeated coolings in ice, and the homogenate, after threefold freezing and thawing, is subjected to sonication, said process being characterized in that it further comprises the successive operations of:

a) subjecting the sonicated matter to a previous centrifugation at 3000 x g according to the following scheme:

```
          Previous centrifugation at 3000 x g

     ┌─ Supernatant (1)    · Sediment (1)        ┌─ homonogeniz-
     │                                           │  ation, soni-
Pool │   Supernatant (2)      Sediment (2)       │  cation, cen-
     │                                           └─ trif. 3000xg
     └─ Supernatant (3)       Sediment (3)   (to be discarded)
```

in which the sediment (1) from the first precentrifugation, after homogenization, sonication and further centrifugation at 3000 x g gives rise to the supernatant (2) and to the sediment (2) and the latter, subjected to the same procedure, forms the sediment (3) that is discarded, and the supernatant (3) that is added to the supernatants (1) and (2), to form a pool,

b) centrifuging said pool of the supernatants (1), (2) and (3) at 100,000 x g and concentrating the supernatant,

c) introducing, after activity test, the supernatant into an isoelectric focussing (IEF) column and collecting the peak fractions, checking their activities, and

d) increasing the purification degree by treatment on a chromatographic column.

2. A process according to claim 1 wherein said sonication is carried out at 0.9 kc in ice and the centrifugation of said pool of supernatants at 100,000 x g is kept going for 60', the whole procedure being carried out at 4°C in a sterilized environment.

3. A process according to claim 1 or 2, wherein the concentration of the final supernatant is carried out by filtering through sucrose and/or other filtering aids such as polyacrylamide or Sephadex, and the isoelectric focussing is performed at a pH of 3.5-10, the final purification step by chromatography being performed on a DEAE A (Sepharose) column.

4. Antigenic protein products (TLP) obtainable according to the process claimed in each one of the claims 1-3, said products consisting of lipoglycoproteins having the following properties:

14

a) sizes between 4.81 and 6.1 nm (48.1 and 61 Angstrom)
b) presence of a lipidic group
c) solubility, and
d) isoelectric point lower than 7.0.

**Patentansprüche**

1. Verfahren zum Extrahieren aus humanen neoplastischen Zellen von Membranantigenenen, die immunogenen Eigenschaften aufweisen und eine verzoegerte Hypersensibilitaet und spezifische Blastization von Lymphozyten zu veranlassen vermoegen, wobei das neoplastische Gewebe, nach Beseitigung des nekrotisches Stoffes und Waschen in einer Phosphatpuffer-Loesung (PBS:Phosphate Buffer Solution), feinst zerkleinert und durch wiederholte Kuehlung im Eis homogenisiert wird und das Homogenat, nach dreimaligem Gefrieren und Auftauen, der Sonikation (Schallwellenaussetzung) unterworfen wird, dadurch gekennzeichent, dass es ausserdem die folgenden Verfahrensschritte aufweist:

   a) Unterwerfung des durch Sonikations behandelten Stoffes einer Vor-Zentrifugation bei 3000 x g gemaess dem folgenden Schema:

```
         Vor-Zentrifugation bei 3000 x g

    ┌  Supernatant (1)    Sediment (1)   ┌ Homogenisierung
    │                                     │
Pool│  Supernatant (2)    Sediment (2)   │ Sonikation,   Zen-
    │                                     │ trifugation bei
    │                                     │ 3000 x g
    └  Supernatant (2)    Sediment (3)    (zu beseitigen)
```

   worin das Sediment (1) aus der ersten Vor-Zentrifugation, nach Homogenisierung, Sonikation und weiterer Zentrifugation bei 3000 x g zum Supernatant (2) und zum Sediment (2) fuehrt und dieses, nach der gleichen Behandlung, das Sediment (3) bildet, das beseitigt wird, und das Supernatant (3), die zu den Supernatanten (1) und (2) zugegeben wird, um das Pool zu bilden,

   b) Zentrifugation dieses aus den Supernatanten (1), (2) und (3) bestehenden Pools bei 100.000 x g und Konzentration des Supernatants,

   c) Einfuehrung, nach Aktivitaets-Test, des Supernatants in eine isoelektrische Fokussierungs-Saeule (IEF) und Entnahme der Peak-Fraktionen, Untersuchung deren Aktivitaet und

   d) Erhoehung des Reinheitsgrades durch Behandlung auf einer chromatographischen Saeule.

2. Verfahren nach Anspruch 1, worin die vorgenannte Sonikation bei 0,9 kHz im Eis durchgefuehrt wird und die Zentrifugation des vorgenannten Pools von Supernatanten bei 100.000 x g 60' dauert, wobei das ganze Verfahren bei 4°C in steriler Umgebung durchgefuehrt wird.

3. Verfahren nach den Anspruechen 1 oder 2, dadurch gekennzeichnet, dass die Konzentration des Endsupernatants vermittels einer Filtration durch Sukrose und/oder anderes Filtermittel, wie Polyakrylamid oder Sephadex, durchgefuehrt wird und die isoelektrische Fokussierung bei pH-Wert 3,5-10 stattfindet, wobei der Endreinigungsschritt auf einer DEAE A (Sepharose) Saeule durchgefuehrt wird.

4. Antigenische Protein-Produkte (TLP) erhaltenden nach dem in je einem der Ansprueche 1 bis 3 beanspruchten Verfahren, die aus Lipoglykoproteinen bestehen, die folgenden Eigenschaften aufweisen:

   a) Groesse zwischen 4,81 und 6,1 nm (48,1 und 61 Angstrom);
   b) Vorhandensein einer lipidischen Gruppe;

c) Loeslichkeit und
d) isoelektrischer Punkt unter 7,0.

**Revendications**

1. Procédé d'extraction, à partir de cellules néoplastiques humaines, d'antigènes membranaires ayant des propriétés immunogènes et capables d'induire l'hypersensibilité retardée et la blastisation spécifique des lymphocytes, dans lequel le tissu néoplastique, après l'élimination du matériel nécrotique et le lavage dans une solution tampon au phosphate (PBS: Phospahte Buffer Solution), est réduit en miettes et homogéneisé par plusieurs refroidissements sur glace et l'homogénat, après trois congélations et décongélations, est soumis à sonication, ledit procédé étant caractérisé en ce qu'il comprend les opérations successives de:
    a) sousmission du matériel soniqué à une pré-centrifugation à 3000 x g selon le schèma suivant:

Pré-centrifugation à 3000 x g

Surflottant (1)  Sédiment (1)  [homogéneisation, sonication, centrifugation à 3000 x g

Pool Surflottant (2)  Sédiment (2)

Surflottant (3)  Sédiment (3)  (à éliminer),

dans lequel le sédiment (1) de la première pré-centrifugation, après homogéneisation, sonication et une autre centrifugation à 3000 x g, produit le surflottant (2) et le sédiment (2) et ce dernier, soumis au même traitement, produit le sédiment (3), qui est éliminé, et le surflottant (3), qui est ajouté aux surflottants (1) et (2) pour former un pool;
    b) centrifugation dudit pool des surflottants (1), (2) et (3) à 100.000 x g et concentration du surflottant;
    c) introduction, après test d'activité, du surflottant dans une colonne de focalisation isoélectrique (IEF) et collection des fractions de peak, contrôle de leur activité et
    d) augmentation du degré de pureté par traitement sur une colonne chromatographique.

2. Procédé selon la revendication 1, dans lequel ladite sonication est effectuée à 0,9 kHz dans glace et la centrifugation dudit pool des surflottants à 100.000 x g est continuée pour 60', le procédé entier étant realisé à 4°C dans un milieu stérilisé.

3. Procédé selon la revendication 1 ou 2, dans lequel la concentration du surflottant fin est réalisée par filtration à travers du saccharose et/ou un autre moyens de filtration, comme polyacrylamide ou Sephadex, et la focalisation isoélectrique est effectuée à un valeur pH entre 3,5 et 10, la phase de dépuration finale par chromatographie étant réalisée sur une colonne DEAE A (Sepharose).

4. Produits proteiques antigèniques (TLP) réalisables selon le procédé revendiqué dans chacune des revendications 1-3, lesdits produits consistant de lipoglycoproteines ayant les propriétés suivantes:
    a) dimension entre 4,81 et 6,1 nm (48,1 et 61 Angstrom);
    b) présence d'un group lipidique;
    c) solubilité et
    d) point isoélectrique inférieur à 7,0.